(19) Europäisches Patentamt European Patent Office Office européen des brevets

(11) EP 1 975 230 A1

(12) EUROPEAN PATENT APPLICATION

(43) Date of publication:
**01.10.2008 Bulletin 2008/40**

(21) Application number: **07090063.4**

(22) Date of filing: **30.03.2007**

(51) Int Cl.:
***C12N 11/10*** (2006.01) ***C12N 11/14*** (2006.01)
***C07K 17/10*** (2006.01) ***C07K 17/14*** (2006.01)
***C12Q 1/54*** (2006.01) ***G01N 33/66*** (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Capsulution Nanoscience AG**
**12489 Berlin (DE)**

(72) Inventor: **Nolte, Marc**
**10247 Berlin (DE)**

(74) Representative: **Zimmermann & Partner**
**Postfach 330 920**
**80069 München (DE)**

(54) **Method for forming glucose sensing micro-particles, use of micro-particles, and kit**

(57) A method for forming glucose sensing micro-particles is provided. The method comprises the steps of (a) providing a suspension comprising hydrogel micro-particles, the hydrogel micro-particles comprising a covalently cross-linked or ionically cross-linked hydrogel matrix which comprises functional groups; (b) adding a reagents to the suspension to activate the functional groups of the hydrogel micro-particles; and (c), after having activated the functional groups, adding a glucose-binding lectin, such as concanavalin A in pure or modified form, a stabilised glucose-specific enzyme, an appropriate molecule from aptamer or spiegelmer classes, or a mixture thereof to the suspension to link the glucose-binding lectin, stabilised glucose-specific enzyme, or molecule from aptamer or spiegelmer classes covalently to the activated functional groups.

*Fig. 2*

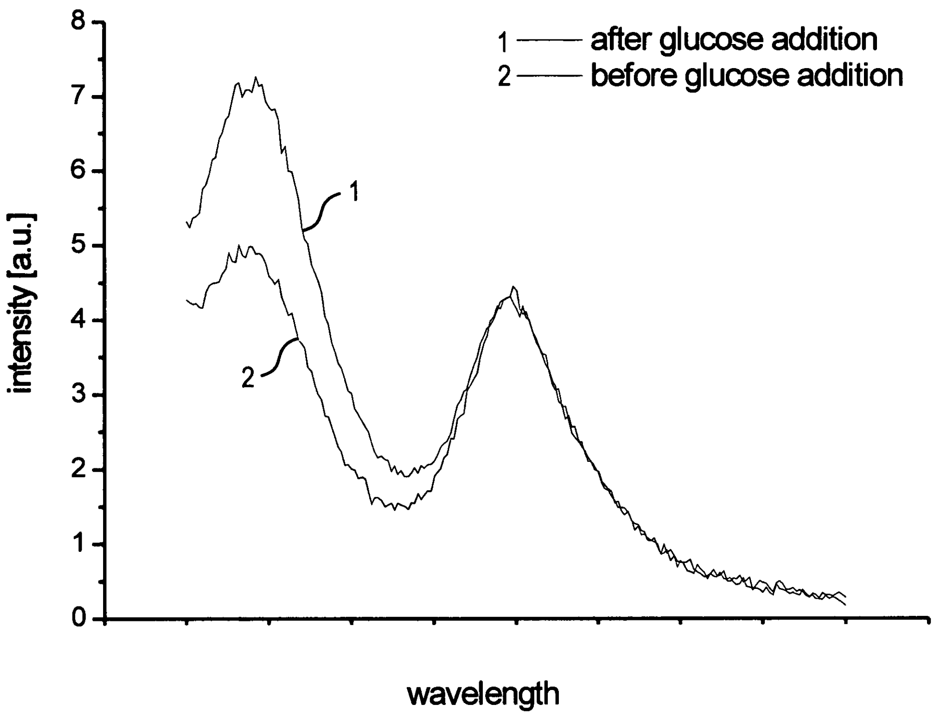




Printed by Jouve, 75001 PARIS (FR)

## Description

The invention pertains to a method for forming glucose sensitive micro-particles and in particular to glucose sensitive micro-particles formed by hydrogel particles comprising dextran and a glucose-binding lectin, such as concanavalin A, a stabilised enzyme, an aptamer, or spiegelmer.

BACKGROUND OF THE INVENTION

For glucose sensing a sensitive system, comprising two components is widely used. The components are macromolecular dextran and concanavalin A (Con A), a glucoses-specific lectin. Concanavalin A can reversibly bind four glucose residues due to its affinity to glucose and thus forming a reversible network with dextran. In the presence of glucose, dextran is reversibly displaced by glucose. This competitive binding can be used to form a glucose-sensitive system. For example, a first dye can be covalently coupled to dextran as a donor, and a second dye can be covalently coupled to concanavalin A as an acceptor. The dyes are chosen such that the emission wavelength of the donor overlaps the excitation wavelength of the acceptor to allow a Förster resonance energy transfer, which is also commonly known as fluorescence resonance energy transfer (FRET), between the donor and the acceptor. The energy transfer between the donor and the acceptor strongly depends on the distance between the dyes. By exciting the donor, the emission intensity of the acceptor and/or the donor can be used as a measure to evaluate the distance between both dyes, which allows an estimation of the amount of glucose and dextran bound to concanavalin A.

Commonly, both dyes, which are coupled to dextran and concanavalin A, respectively, are caged in a micro particle having a diameter between 10 and 60 μm. An example is Poly-L-Lysine coated calcium alginate microencapsulated Con A and dextran as for instance described in Russel et al., "A Fluorescence Glucose Assay Using Poly-L-Lysine and Calcium Alginate Microencapsulated TRITC-Succinyl Concanavalin A and FITC-Dextran" IEEE Engineering in Medicine and Biology Society, Vol. 20, No. 6, 1998, pp. 2858 - 2861 and US 6 485 703. Glucose can freely diffuse in and out of these particles, which ensures that the glucose concentration inside the particle changes proportionally to the concentration in a solution in which the particles are suspended. The interaction between glucose and concanavalin A is stronger than the interaction between dextran and concanavalin A. When dextran is displaced by glucose, dextran can more freely diffuse within the particles resulting in an increased average distance between the FITC-dye forming the donor and the TRITC-dye forming the acceptor. This results in an increased emission intensity of the donor since the excitation of the donor cannot be transferred any further radiationless to the acceptor. At the same time, the emission intensity of the acceptor decreases.

When the glucose level in the solution reduces, glucose diffuses out of the particles and allows dextran to form again a complex with concanavalin A, since both molecules remain in the PLL-coated microcapsule and cannot diffuse out. The excitation of the donor can thus increasingly be transferred to the acceptor whereby the detectable emission intensity of the donor reduces.

Another commonly used system for detecting glucose concentration comprises covalently bound concanavalin A to polyethylene glycol (PEG) as for instance described in the above mentioned US 6 485 703, US 2005/0267326 and US 7 166 458.

SUMMARY OF THE INVENTION

According to a first aspect of the invention, a method for forming glucose sensing micro-particles is provided. The method comprises the steps of:

(a) providing a suspension comprising hydrogel micro-particles, the hydrogel micro-particles comprising a covalently cross-linked or ionically cross-linked hydrogel matrix, which comprises functional groups;

(b) adding one or more reagents to the suspension to activate the functional groups of the hydrogel micro-particles; and

(c) after having activated the functional groups, adding a glucose-binding lectin, such as concanavalin A in pure or modified form, a stabilised glucose-specific enzyme, an appropriate molecule from aptamer or spiegelmer classes, or a mixture thereof to the suspension to link them covalently to the activated functional groups.

In the following description, a glucose-binding lectin, such as concanavalin A in pure or modified form, a stabilised glucose-specific enzyme, and an appropriate molecule from aptamer or spiegelmer classes are referred to as glucose-specific molecule.

The functional groups of the hydrogel micro-particles or its hydrogel matrix are activated before the glucose-specific molecule is added. The "non-activated" glucose-specific molecule therefore only reacts with the functional groups of the hydrogel matrix. Unlike previous attempts to form hydrogel micro-particles by copolymerisation in the presence of glucose-specific molecule such as concanavalin A, the micro-particles are formed and cross-linked before glucose-specific molecule is covalently bound to the polymer backbone of the hydrogel matrix according to an aspect of the invention.

It has been observed by the inventors that by avoiding activation of the glucose-specific molecule the functionality of the glucose-specific molecule can be maintained. It is believed, without wishing to be tight to theory, that copolymerisation in the presence of an glucose-specific molecule results in a partial cross-linking of the glucose-specific molecule, which reduces the ability of the glucose-specific molecule to bind glucose. It is assumed that internal cross-linking of the glucose-specific molecule also occurs when it is activated by an appropriate reagent since many glucose-specific molecules comprise functional groups, which are similar to functional groups of the hydrogel polymer matrix. Therefore, the glucose-specific molecule is added to the suspension without any preceding activation of glucose-specific molecule.

To ensure that the glucose-specific molecule is not activated by the reagents added to the suspension a washing step such as filtration or centrifugation can be performed after activation of the functional groups of the hydrogel matrix.

The glucose-specific molecule is conjugated with a dye, which forms together with a dye conjugated to dextran a FRET-pair. In many applications, the glucose-specific molecule is a glucose-binding lectin such as concanavalin A.

According to an embodiment, the functional groups of the hydrogel matrix may comprise carboxyl groups, which can be for instance activated by *N*-Ethyl-*N'*-(3-dimethylaminopropyl)carbodiimide hydrochloride and N-Hydroxysulfosuccinimide sodium salt (EDAC/NHS), conversion of the carboxylic acids to anhydrids, and carboxylic acid chlorides. Harsh reaction conditions do not cause problems as the sensitive protein and the dye conjugated to the glucose-specific molecule are not exposed.

For forming the hydrogel micro-particles prior to bind glucose-specific molecule, a radical polymerisation of monomers can be for instance used. The monomers might comprise functional groups suitable to bind the glucose-specific molecule. Alternatively, the respective functional groups are added prior to polymerisation. It is also possible to form the hydrogel micro-particles by ionically cross-linking of polyelectrolytes using appropriate multivalent counter-ions. In some embodiments, divalent counter-ions are used.

Dextran conjugated with the other dye is typically added after covalently coupling the glucose-specific molecule.

## BRIEF DESCRIPTION OF THE DRAWINGS

A full and enabling disclosure of the present invention, including the best mode thereof, to one of ordinary skill in the art, is set forth more particularly in the remainder of the specification, including reference to the accompanying figures. Therein:

Figures 1A and 1B show alginate hydrogel beads with covalently coupled concanavalin A (Figure 1A) and uncoupled reference (Figure 1B).

Figure 2 shows an increase of the donor signal due to glucose addition to alginate beads with covalently coupled concanavalin A.

Figure 3 shows polyacrylate hydrogel beads with covalently coupled concanavalin A.

Figures 4A to 4C shows fluorescence recovery after photo bleaching (FRAP) experiments. Figure 4A shows particles with a photo bleached spot 3 min after bleaching. The diffusion of concanavalin A is hindered due to the coupling. Uncoupled concanavalin A in a similar bead just after bleaching is shown in Figure 4B and after 3 minutes in Figure 4C indicating the mobility of concanavalin A..

Figure 5 shows an increase of the donor signal due to glucose addition to polyacrylate beads with covalently coupled concanavalin A.

## DESCRIPTION OF THE PREFERRED EMBODIMENT

For purpose of promoting an understanding of the principles of the invention, reference will now be made to the preferred embodiment illustrated in the drawings and specific language will be used to describe the same. It will nevertheless be understood that no limitation of the scope of the invention is thereby intended, such alterations and further modifications in the illustrated device and/or method, and such further applications of the principles of the invention as illustrated therein being contemplated as would normally occur now or in future to one skilled in the art to which the invention relates.

An aspect of the present invention as described in this specification is the covalent coupling of a glucose-specific molecule to hydrogel particles. This allows for a caging of the molecule without requiring an additional cage around the particle and therefore avoids the leakage of the concanavalin A. As dextran is adsorbed quantitatively and therefore does not diffuse out of the particles, this is a fast way to fulfil the conditions of caging dextran and concanavalin A within hydrogel particles.

In the following, the invention is described in connection with glucose-binding lectin and specifically, in connection with concanavalin A. Concanavalin A is conjugated with an appropriate dye to form a FRET pair with another appropriate dye conjugated to dextran. A skilled person will appreciate that a variety of dyes are suitable to form FRET dye pairs. An example is FITC and TRITC. It is intended that the invention is not restricted to specific dyes.

Typically, the micro-particles can have a size between about 500 nm and about 60 μm to about 100 μm. This size range has proved to be of advantage for many sensing application since the micro-particles are small enough to be finely dispersed in the measuring environment while at the same time large enough to give sufficient fluorescence intensity.

For coupling concanavalin A to the hydrogel particles, different functional entities can be used, e.g. the coupling of amines to carboxyl groups of the hydrogel using EDAC/NHS, the conversion of an amine of the protein to an azide, and further reactions or reactions of the "click chemistry" after introduction of the respective groups. The reaction conditions have to be chosen in a way that the activity of the protein is maintained. In some embodiments, concanavalin A is coupled to the hydrogel micro-particles in the presence of glucose to ensure that the glucose-specific binding sites of concanavalin A are occupied by glucose and cannot react with the activated functional groups of the hydrogel micro-particles. This further also maintains the glucose-specific binding capacity of concanavalin A.

Typically, the hydrogel particles are formed before concanavalin A is covalently bound to the polymer matrix of the hydrogel particles so that the concanavalin A can be bound to the polymers, which are already cross-linked or ionically linked. Examples are polyacrylates, polymethacrylates, polyvinylpyrrolidon, and polyalcohols.

Hydrogel particles can be manufactured in different ways. One option is a radical polymerisation of monomers. The monomers may naturally carry a functional group onto which the concanavalin A can be bound later. Alternatively, the functional group can be added to the monomers before polymerisation.

As an example, hydrogel particles can be formed by radical polymerisation using

(A) acrylic monomers having a carboxyl group such as methacrylic acid and acrylic acid,

(B) non-ionic monomers, such as acrylamide, poly(ethylene glycol) acrylate or 2-hydroxyethyl methacrylate, and

(C) a cross-linking agent such as bisacrylates, for example *N*,*N'*-methylenebisacrylamide or poly(ethylene glycol) dimethacrylate.

The radical polymerisation is typically carried out in an emulsion or concentrated emulsion to form the micro-particles. Alternatively, only acrylic monomers (A) and a cross-linking agent (B) without additional non-ionic monomers can be used.

If non-acryl monomers such as polysugars, for example alginate, dextran and hyaluronic acid, are to be polymerised, the non-acryl monomers are acrylated using for example glycidylmethacrylate and at least an acrylate selected from the group comprises acrylates of the above group (A), (B), (C), and mixtures thereof. The acrylated monomers are then polymerised by radical polymerisation in an emulsion or concentrated emulsion.

An example of the group (A) compounds is:

**Methacrylic acid**

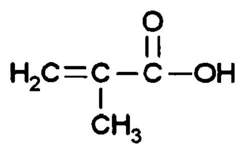

Examples of group (B) compounds are:

Poly(ethylene glycol) acrylate

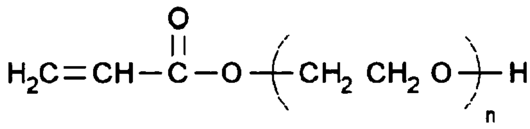

2-Hydroxyethyl methacrylate

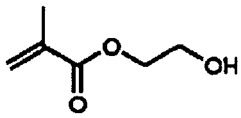

Glycidyl Methacrylate

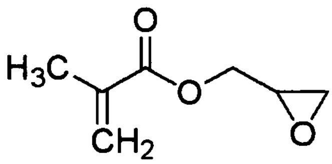

Examples of the group (C) compounds are:

*N*,*N'*-Methylenebisacrylamide

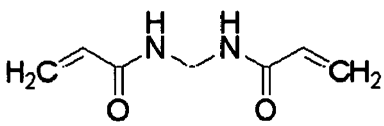

Poly(ethylene glycol) dimethacrylate

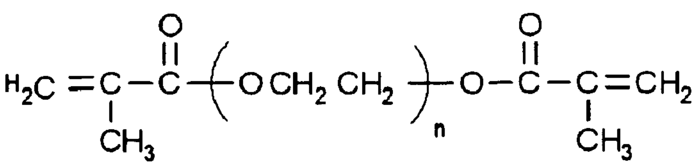

Different to radical polymerisation is the formation of hydrogel particles by ionically linking polyelectrolytes using

multivalent counter-ions. For example, alginate can ionically be cross-linked with group II cations such as calcium, which results in a micro-particle gelation of alginate.

Two hydrogels, which were used as examples, are ionically cross-linked alginate and covalently cross-linked acrylate hydrogels. Both of the examples offer the advantage of a quantitative uptake of dextran, which will therefore not diffuse out of the hydrogel even without being physically cross-linked to the polymer matrix.

The acrylate hydrogels for example can contain acrylamide and methacrylic acid as linear components for the hydrogel formation and *N,N'*-Methylenbisacrylamide as cross-linking agent. Different ratios of acrylamide and methacrylic acid can be used (e.g. 1/1, 3/1 15/1) all with a degree of cross-linking of 2.5% having a total acrylate concentration of 16.4% in the reactive solution. The particles can be formed using a concentrated emulsion polymerisation with ammoniumperoxidisulfate as starter and *N,N,N',N'*-tetramethylethylendiamine as catalyst. The resulting particles are strongly swelling in water and have a size distribution in the range mentioned above.

The formation of the hydrogel micro-particles including any cross-linking should be completed before concanavalin A is covalently bound to the polymer matrix. For maintaining the binding activity of concanavalin A and the fluorescence capability of the conjugated dye, the functional groups of the polymer matrix only are activated without exposing concanavalin A to the reagents which is used for activation. For example, the covalently or ionically cross-linked hydrogel micro-particles can be washed with a buffer solution to adjust the pH within the micro-particles. The activation is performed using e.g. *N*-Ethyl-*N'*-(3-dimethylaminopropyl)carbodiimide hydrochloride and *N*-Hydroxysulfosuccinimide sodium salt (EDAC/NHS). The reagents are incubated with the hydrogel micro-particles and subsequently washed away using filtration or centrifugation so that no activation reagents remain in the micro-particle suspension.

After activation, concanavalin A is added to the micro-particle suspension and binds to the activated functional groups of the polymer matrix, thereby avoiding a cross-linking between concanavalin A molecules. For example, concanavalin A can be dissolved in a desired buffer and then added to a suspension or sediment of the activated hydrogel micro-particles. The pH-value of the buffer for dissolving concanavalin A and the pH-value of the suspension should be adjusted to ensure that concanavalin A can penetrate the hydrogel micro-particles. For hydrogel micro-particles being formed by polymerisation of acrylates the suspension should be at least slightly acidic with a pH-value less at about 6. Typically, the pH-value is adjusted in this case to be higher than about 5 to avoid exposing concanavalin A to strong acidic environment. On the other hand, if the hydrogel micro-particles are formed by cross-linked alginate, the suspension can be either acidic or basic. Typically, the pH-value is adjusted to be outside of a range between about 6 and about 7.4 since concanavalin A is in this range only slightly soluble. For acidic environment, the pH-value can be adjusted to be between about 5 and 6 and for basic environment the pH-value can be in the range between 7.4 and about 8.

If concanavalin A is dissolved in a buffer, which also has functional groups similar to concanavalin A, the solution should be dialysed against another suitable buffer. For example, concanavalin A is commercially available in a solution of a TRIS-buffer (trishydroxymethylaminomethane) carrying also amino groups which will bind to the activated carboxyl groups of the polymer matrix. To avoid the binding of TRIS to the polymer matrix, the concanavalin A solution can be for instance dialysed against a borate buffer. The pH-value of TRIS and borate buffer is slightly basic and concanavalin A exhibits in such an environment a negative net charge. This would prevent the incorporation of concanavalin A into hydrogel micro-particles formed by acrylates, which also have a negative net charge. This problem, however, can be overcome by ensuring that the buffer used in the acidic suspension of the polyacrylate micro-particles can substantially buffer the pH-value even when the basic concanavalin A solution is added. For instance, the suspension may contain a sufficient amount of buffer for maintaining the acidic environment. By adding the concanavalin A solution to the suspension the net charge of the concanavalin A is instantly changed thereby avoiding an aggregation of concanavalin A. During mixing of the concanavalin A solution with the micro-particle suspension, stirring or shaking improves mixing and further reduces aggregation of concanavalin A.

After a sufficient incubation time the non-bound concanavalin A is washed away using for example water or borate buffer.

Dextran can be incorporated into the micro-particles either before or after binding of concanavalin A. If functional groups were added to the dextran, e.g. for the coupling of the dye which would also react with the activated groups of the hydrogel, dextran is added after the covalently coupling of concanavalin A.

If desired, the micro-particles can finally be coated by one or more layers of alternately charged polyelectrolytes, which can improve the stability of the micro-particles.

The described particles can be embedded in bigger structures e.g. in implants which are implanted allowing a measurement of the blood glucose level in vivo. It is also possible to use the micro-particles for glucose-sensing applications in non-medical applications such as food production.

The above-described approach of caging concanavalin A and dextran in the micro-particles has been proved to produce stable micro-particles with a sufficiently strong fluorescence response to variation of the glucose concentration. The micro-particles allow the reversible formation of a complex between dextran and concanavalin A depending on the glucose concentration. In particular, a continuous forming and dissolving of the complex ensures that both components remain in the micro-particles and cannot diffuse away too far from each other.

The hydrogel micro-particles formed by the above method provides a variety of advantages such as:

(a) Dextran and concanavalin A can diffuse within the hydrogel micro-particles.

(b) Glucose can readily diffuse in and out of the hydrogel micro-particles.

(c) At least one of the components (typically dextran) can diffuse freely in the hydrogel to form and dissolve the complex. Alternatively, both compounds can be bound to the polymer matrix.

(d) Functional entities are provided to couple covalently one or both components to the polymer matrix.

(e) The binding capacity of concanavalin A and the fluorescence capacity of the conjugated dye are less affected by the covalently coupling to the polymer matrix than in commonly used copolymerisation approaches

In the following, specific examples are described from which further aspects and advantages can be drawn.

**Example 1: Coupling of concanavalin A to alginate beads**

2.5 ml of alginate beads (micro-particles) of a 4.23 vol% solution were washed three times with borate buffer. To activate the micro-particles for coupling of the protein, 105 μl of a 10 mg/ml solution of Sulfo-NHS in 10 mM borate buffer at pH 8 and 28.5 μl of a 100 mg/ml solution of EDAC in 10 mM borate buffer at pH 8 were added. This results in an activation of about 15% of the carboxyl groups of the alginate. After 30 min, the micro-particles were separated by centrifugation and washed twice with water to avoid reactions of the protein with unreacted NHS and EDAC. The particles were centrifuged again and 950 μl of a 5.9 g/l solution of concanavalin A in 10 mM borate buffer at pH 8 was added and incubated over night. The particles were washed until no concanavalin A could be detected in the supernatant using fluorescence spectroscopy.

The concentration of concanavalin A in the particles is approximately 6.5 g/l. After adding dextran to the particle suspension, the suspension was washed again and filled up with 10 mM TRIS buffer at pH 7.4 to adjust the particle concentration in the suspension. The glucose response is measured by diluting 25μl of the bead suspension with 1975 μl of 10 mM TRIS buffer at pH 7.4. The fluorescence spectrum of the suspension is recorded using an excitation of the donor. The emission of the donor and acceptor were measured, respectively. 14 μl of a 1 M glucose solution is added and the spectrum is recorded again. The difference in intensity gives the glucose response using the formula:

$$\text{Glucose response [\%]} = ((\text{Intensität}_{100mg} - \text{Intensität}_{0mg}) / \text{Intensität}_{0mg}) * 100$$

The glucose response was measured and calculated to be 45%.

Figure 1A shows a microscopy image of an alginate hydrogel beads suspension with covalently coupled concanavalin A whereas Figure 1B shows an uncoupled reference. The gain for the image shown in Figure 1B is set to about 5.5 so that the intensity is comparable with the intensity of the image shown in Figure 1A. This also shows that without covalently binding only very few concanavalin A is incorporated into the micro-particles.

The increase of the donor signal with respect to the addition of glucose as described above is shown in Figure 2, wherein curve 1 shows the emission of the donor and acceptor after glucose addition and curve 2 before glucose addition..

**Example 2: Coupling of concanavalin A to polyacrylate beads**

2.5 ml of polyacrylate beads (micro-particles) having a acrylamide/methacrylic acid ration of 1/1 and a concentration of 22 vol% were washed three times with MES (2-(*N*-morpholino)ethanesulfonic acid) buffer. To activate the particles for coupling the protein, 53.4 μl of a 100 mg/ml solution of Sulfo-NHS in 10 mM MES buffer at pH 5.6 and 19.60 μl of a 100 mg/ml solution of EDAC in 10 mM MES buffer at pH 5.6 were added. This is an activation of about 100% of the carboxyl groups of the polyacrylate beads. After 30 min, the particles were separated by centrifugation and washed twice with water to avoid reactions of the protein with unreacted NHS and EDAC. The particles were centrifuged again and resuspended in 2.5 ml with 50 mM MES buffer at pH 5.6. 1250 μl of a 5.6g/l solution of concanavalin A in 10 mM borate buffer at pH 8 was added and incubated over night. Due to the higher volume and the higher buffer concentration

in the particle suspension, the pH of the suspension does not change much when the concanavalin A solution with the borate buffer is added. The micro-particles were washed with 10 mM borate buffer at pH 8 until no concanavalin A could be detected in the supernatant using fluorescence spectroscopy.

An example of polyacrylate hydrogel beads with covalently coupled concanavalin A is shown in a confocal fluorescence microscopy image shown in Figure 3.

For verification of the concanavalin A coupling to the polyacrylate beads, fluorescence recovery-after-photobleaching-experiments have been performed. Different to the case were concanavalin A was not coupled covalently to the polyacrylate beads, the fluorescence of an area of a bead which was bleached using a confocal microscope does not recover due to the restricted diffusion of concanavalin A since it is covalently coupled to the bead. Figure 4A shows a microscopic image of polyacrylate beads with covalently coupled concanavalin A 3 min after bleaching. The arrow in Figure 4A indicates a photo bleached spot, which remains clearly visible after 3 min. This indicates that no recovery takes place due to the restricted diffusion of the bound concanavalin A. Contrary thereto, Figure 4B shows uncoupled concanavalin A in a similar bead just after bleaching and after 3 minutes (Figure 4C). The bleached spot (indicated by an arrow in Figure 4B) is recovered (Figure 4C) due to the possible diffusion of the unbound concanavalin A.

The concentration of concanavalin A in the beads is approximately 22.3 g/l. After adding dextran to the micro-particle suspension, the suspension is washed and filled up with 10 mM TRIS buffer at pH 7.4 to adjust the particle concentration in the suspension. The glucose response is measured by diluting 25μl of the bead suspension with 1975 μl of a 10 mM TRIS buffer at pH 7.4. The fluorescence spectrum of the suspension is recorded using an excitation of the donor. The emission of the donor and acceptor were measured, respectively. 14 μl of a 1 M glucose solution is added and the spectrum is recorded again. The difference in intensity gives the glucose response using the formula:

$$\text{Glucose response [\%]} = ((\text{Intensität}_{100mg} - \text{Intensität}_{0mg}) / \text{Intensität}_{0mg}) * 100$$

The glucose response was measured and calculated to be 15%. The increase of the donor signal with respect to the addition of glucose as described above is shown in Figure 5, wherein curve 3 shows the emission of the donor and acceptor after glucose addition and curve 4 before glucose addition..

**Example 3: Coupling of concanavalin A to alginate beads being cross-linked using Glycidylmethacrylate-modified alginate and N-Vinylpyrolidinone in a radical polymerisation in an emulsion**

Alginate can be acrylated using glycidylmethacrylate and *N*-vinylpyrolidinone and then covalently cross-linked by a radical polymerisation. The thus formed covalently cross-linked alginate beads can be treated as described in example 1 to bind concanavalin A.

The invention also relates to ionically cross-linked or covalently cross-linked alginate micro-particles and covalently cross-linked polyacrylate micro-particles comprising dextran and covalently coupled glucose-binding lectin such as concanavalin A. The invention further pertains to a kit comprising ionically or covalently cross-linked micro-particles; dextran; and a glucose-binding lectin, such as concanavalin A in pure or modified form, a stabilised glucose-specific enzyme, an appropriate molecule from aptamer or spiegelmer classes, or a mixture thereof.

While preferred embodiments have been illustrated and described in detail in the drawings and foregoing description, the same is to be considered as illustrative and not restrictive in character, it being understood that only the preferred embodiment has been shown and described and that all changes and modifications that comes within the spirit of the invention both now or in the future are desired to be protected.

## Claims

**1.** Method for forming glucose sensing micro-particles, comprising the steps of:

(a) providing a suspension comprising hydrogel micro-particles, the hydrogel micro-particles comprising a covalently cross-linked or ionically cross-linked hydrogel matrix, which comprises functional groups;
(b) adding one or more reagents to the suspension to activate the functional groups of the hydrogel micro-particles; and
(c) after having activated the functional groups, adding a glucose-binding lectin, such as concanavalin A in pure or modified form, a stabilised glucose-specific enzyme, an appropriate molecule from aptamer or spiegelmer classes, or a mixture thereof to the suspension to link them covalently to the activated functional groups.

**2.** Method according to claim 1, wherein step (b) further comprises:

(b1) washing the hydrogel micro-particles to remove the reagents.

**3.** Method according to claim 1 or 2, wherein the functional groups comprise at least carboxyl groups.

**4.** Method according to any of the preceding claims, wherein the reagent for activating the functional groups comprises *N*-Ethyl-*N'*-(3-dimethylaminopropyl)carbodiimide hydrochloride and N-Hydroxysulfosuccinimide sodium salt (EDAC/NHS).

**5.** Method according to any of the preceding claims, wherein the step (a) further comprises:

(a1) forming the hydrogel micro-particles by radical polymerisation of monomers.

**6.** Method according to claim 5, wherein the monomer is alginate, which is modified by incorporating a double bond.

**7.** Method according to claim 5, wherein the monomer is an acrylic monomer comprising a carboxyl group which is polymerised by a bisacrylate.

**8.** Method according to claims 1 to 4, wherein the step (a) further comprises:

(a1) forming the hydrogel micro-particles by ionically cross-linking polyelectrolytes using multivalent counter-ions.

**9.** Method according to claim 8, wherein the polyelectrolytes comprises alginate.

**10.** Method according to any of the preceding claims, wherein step (c) comprises:

adding the glucose-binding lectin, stabilised glucose-specific enzyme, the molecule from aptamer or spiegelmer classes, or a mixture thereof to the suspension in the presence of glucose.

**11.** Use of the particles manufactured according to any of the preceding claims for sensing or detecting glucose.

**12.** Kit, comprising:

- ionically cross-linked or covalently cross-linked hydrogel micro-particles;
- a glucose-binding lectin, such as concanavalin A in pure or modified form, a stabilised glucose-specific enzyme, an appropriate molecule from aptamer or spiegelmer classes, or a mixture thereof, and
- dextran.

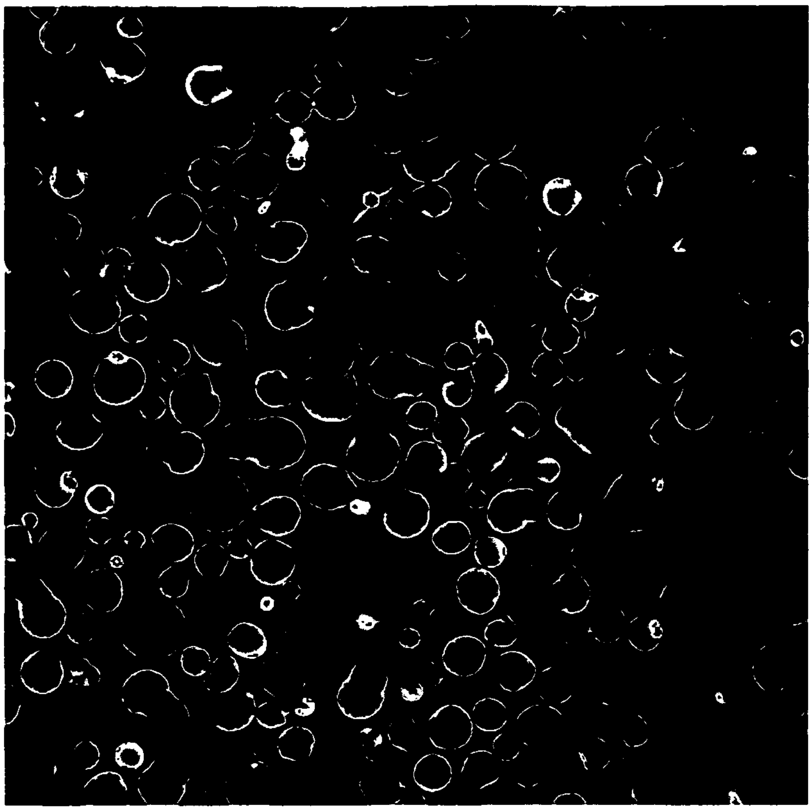

Fig. 1A

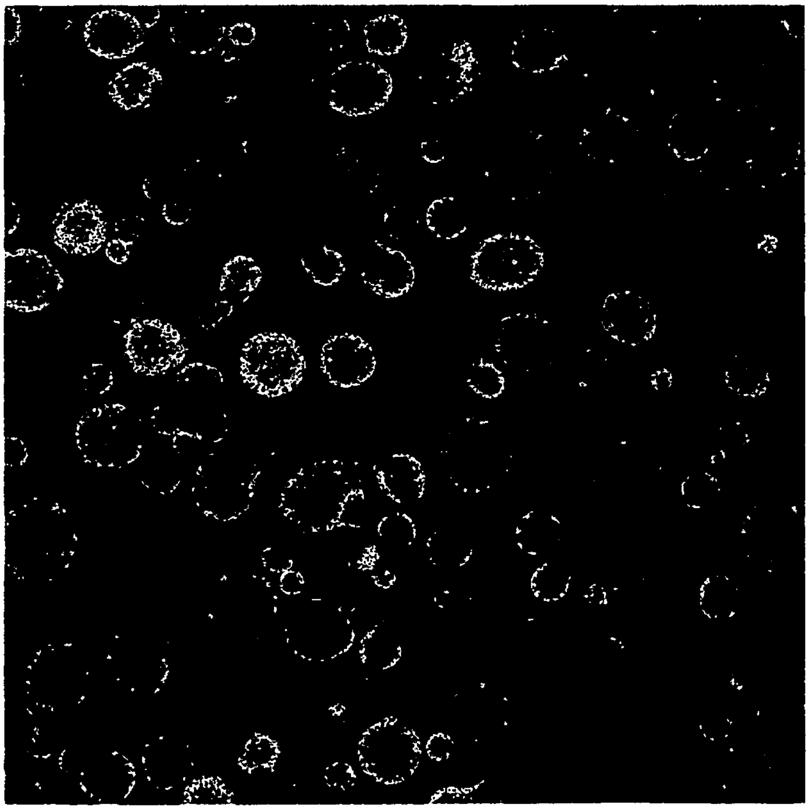

Fig. 1B

*Fig. 2*

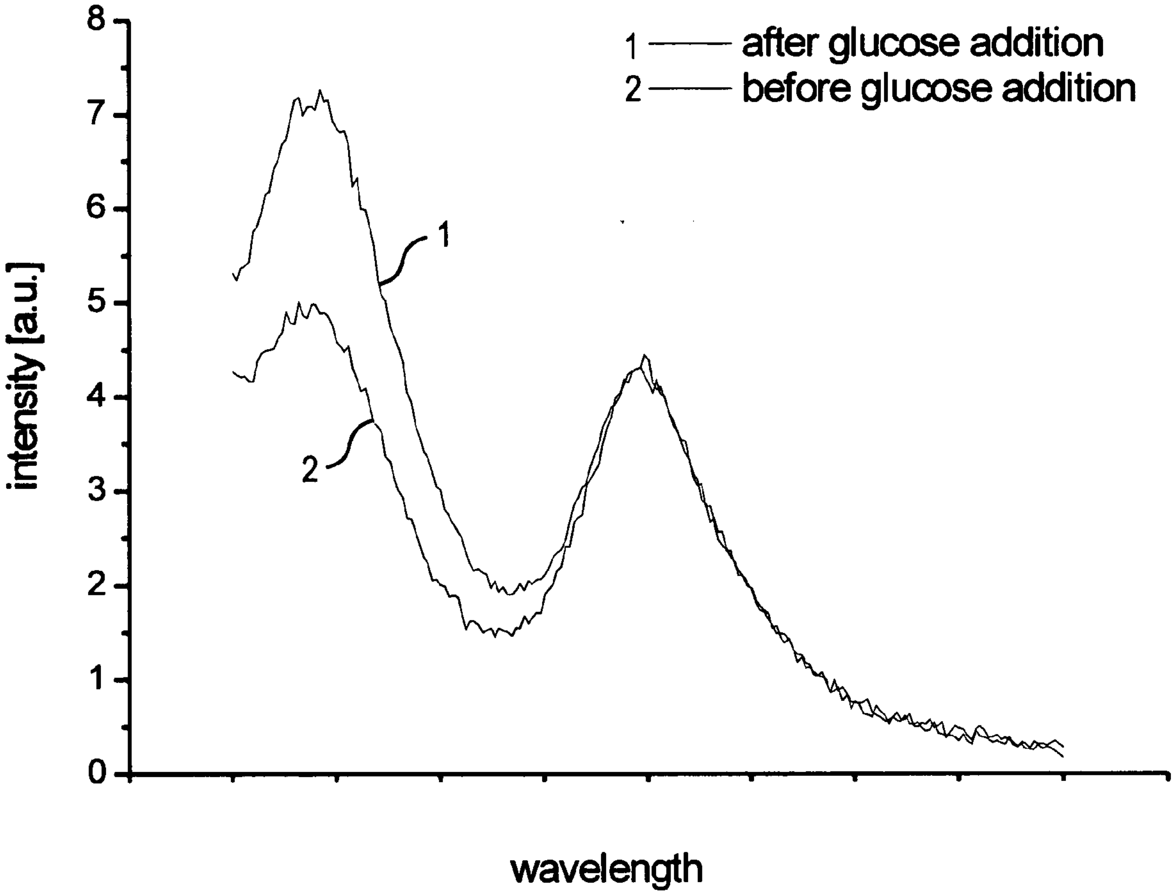
8
7
6
5
4
3
2
1
0
intensity [a.u.]
wavelength
1 —— after glucose addition
2 —— before glucose addition
1
2

Fig. 3

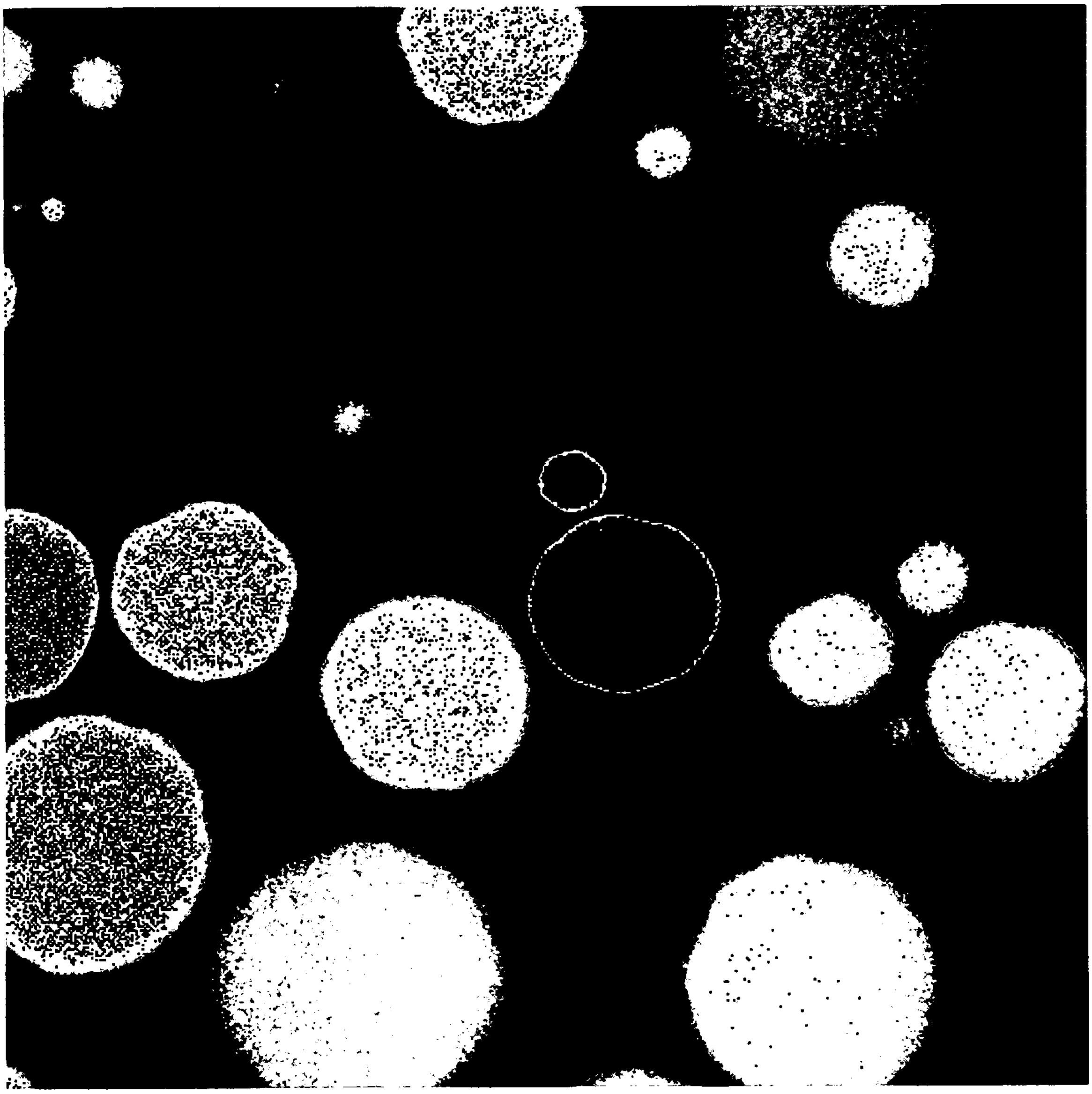

Fig. 4A

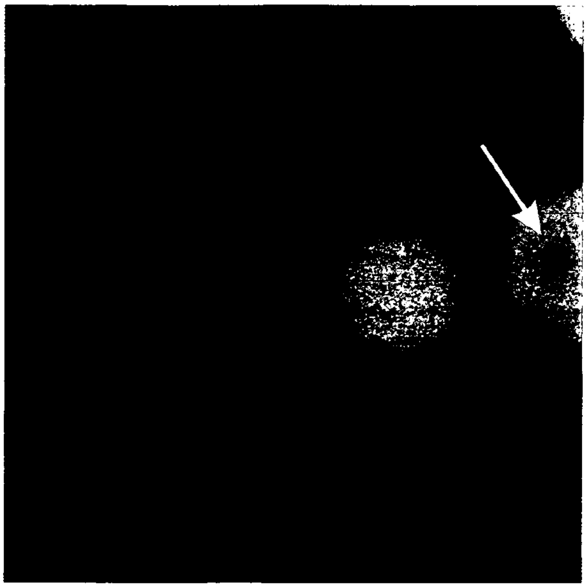

Fig. 4B

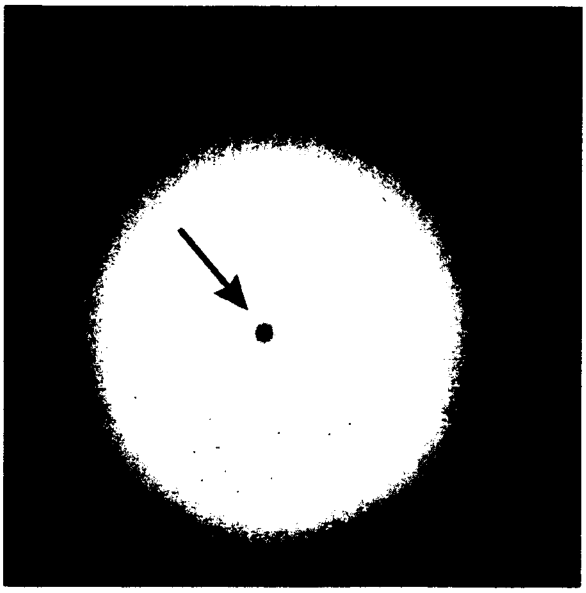

Fig. 4C

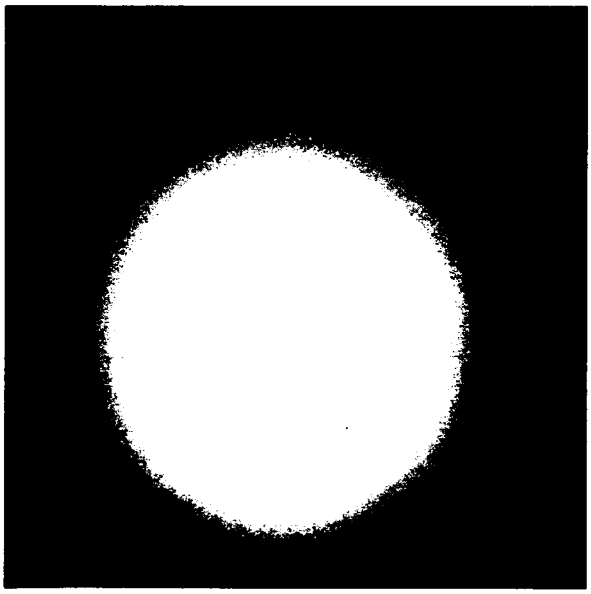

*Fig. 5*

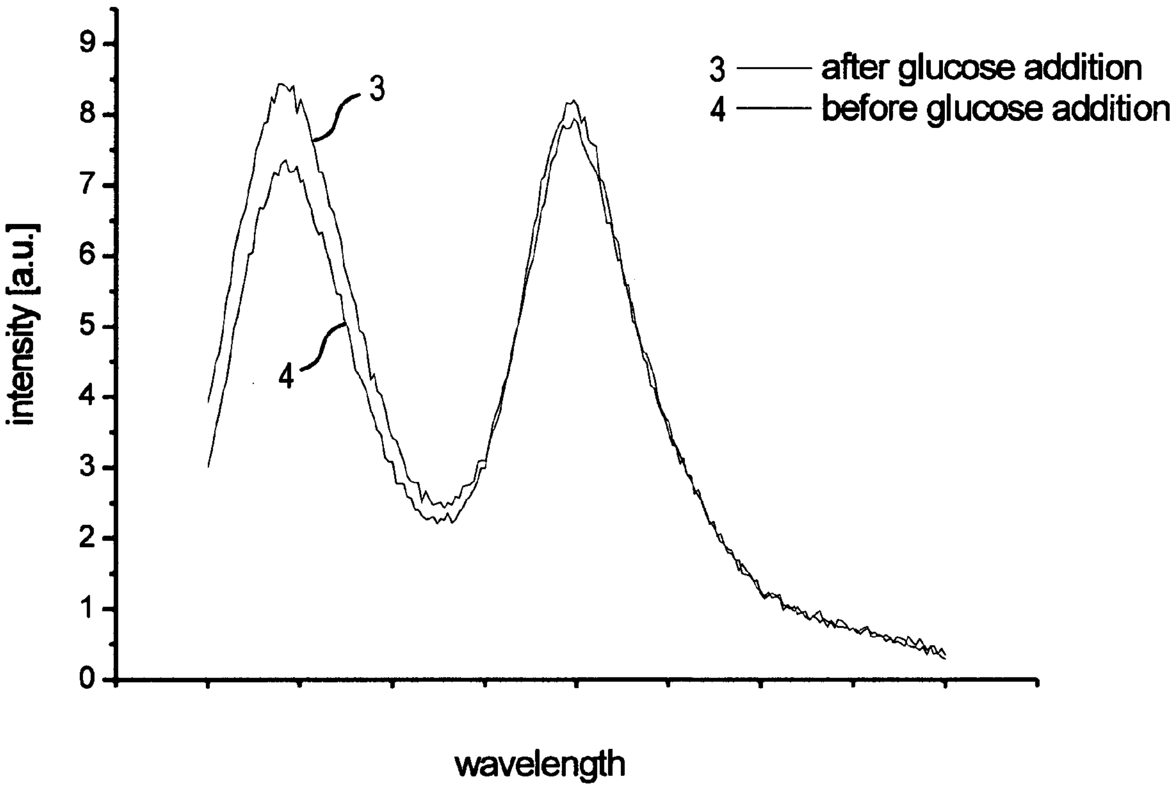
9
8
7
6
5
4
3
2
1
0
intensity [a.u.]
3
4
3 after glucose addition
4 before glucose addition
wavelength

European Patent Office

# EUROPEAN SEARCH REPORT

Application Number
EP 07 09 0063

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GUISEPPI-ELIE A ET AL: "Enzyme microgels in packed-bed bioreactors with downstream amperometric detection using microfabricated interdigitated microsensor electrode arrays." BIOTECHNOLOGY AND BIOENGINEERING 20 NOV 2001, vol. 75, no. 4, 20 November 2001 (2001-11-20), pages 475-484, XP002448576 ISSN: 0006-3592 * the whole document * ----- | 1-11 | INV. C12N11/10 C12N11/14 C07K17/10 C07K17/14 C12Q1/54 G01N33/66 |
| D,X | US 6 485 703 B1 (COTE GERARD L [US] ET AL) 26 November 2002 (2002-11-26) * the whole document * ----- | 12 | |
| A | KRAJEWSKA BARBARA: "Application of chitin- and chitosan-based materials for enzyme immobilizations: a review" ENZYME AND MICROBIAL TECHNOLOGY, STONEHAM, MA, US, vol. 35, no. 2-3, 5 August 2004 (2004-08-05), pages 126-139, XP002444046 ISSN: 0141-0229 * the whole document * ----- | | TECHNICAL FIELDS SEARCHED (IPC) C12N C07K |
| A | HUSSAIN F ET AL: "Glucose sensing based on the intrinsic fluorescence of sol-gel immobilized yeast hexokinase" ANALYTICAL BIOCHEMISTRY, ACADEMIC PRESS, NEW YORK, NY, US, vol. 339, no. 1, 1 April 2005 (2005-04-01), pages 137-143, XP004781309 ISSN: 0003-2697 * the whole document * ----- -/-- | | |

The present search report has been drawn up for all claims

| Place of search | Date of completion of the search | Examiner |
|---|---|---|
| The Hague | 30 August 2007 | Sonnerat, Isabelle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................
& : member of the same patent family, corresponding document

2

EPO FORM 1503 03.82 (P04C01)

European Patent Office

# EUROPEAN SEARCH REPORT

Application Number
EP 07 09 0063

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHINNAYELKA SWETHA ET AL: "Glucose-sensitive nanoassemblies comprising affinity-binding complexes trapped in fuzzy microshells." JOURNAL OF FLUORESCENCE SEP 2004, vol. 14, no. 5, September 2004 (2004-09), pages 585-595, XP002448577 ISSN: 1053-0509 * the whole document * ----- | | |
| A | PICKUP J C ET AL: "Fluorescence-based glucose sensors" BIOSENSORS & BIOELECTRONICS, ELSEVIER SCIENCE PUBLISHERS, BARKING, GB, vol. 20, no. 12, 15 June 2005 (2005-06-15), pages 2555-2565, XP004861166 ISSN: 0956-5663 * the whole document * ----- | | |
| | | | TECHNICAL FIELDS SEARCHED (IPC) |

The present search report has been drawn up for all claims

| Place of search | Date of completion of the search | Examiner |
|---|---|---|
| The Hague | 30 August 2007 | Sonnerat, Isabelle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO.

EP 07 09 0063

This annex lists the patent family members relating to the patent documents cited in the above-mentioned European search report. The members are as contained in the European Patent Office EDP file on
The European Patent Office is in no way liable for these particulars which are merely given for the purpose of information.

30-08-2007

| Patent document cited in search report | Publication date | Patent family member(s) | Publication date |
|---|---|---|---|
| US 6485703 B1 | 26-11-2002 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6485703 B
- US 20050267326 A
- US 7166458 B

### Non-patent literature cited in the description

- **RUSSEL et al.** A Fluorescence Glucose Assay Using Poly-L-Lysine and Calcium Alginate Microencapsulated TRITC-Succinyl Concanavalin A and FITC-Dextran. *IEEE Engineering in Medicine and Biology Society,* 1998, vol. 20 (6), 2858-2861